# EUROPEAN PATENT APPLICATION

(11) **EP 2 559 749 A1**
(43) Date of publication of application: **20.02.2013**
(21) Application number: 10849870.0
(22) Date of filing: 28.12.2010
(51) Int. Cl.: C11D 17/08, A61K 8/34, A61K 8/37, A61K 8/42, A61K 8/46, A61Q 5/02, A61Q 19/10, C11D 1/29, C11D 1/52, C11D 1/88, C11D 3/20

(54) **CONCENTRATED LIQUID DETERGENT COMPOSITION AND PROCESS FOR PRODUCTION THEREOF**

(30) Priority: 12.04.2010 JP 2010091799
(71) Applicant: Shiseido Company, Ltd., Tokyo 104-0061 (JP)
(72) Inventor: KINOSHITA Koichi, Yokohama-shi Kanagawa 224-8558 (JP); KUROKAWA Kenji, Yokohama-shi Kanagawa 224-8558 (JP)
(74) Representative: Henkel, Breuer & Partner
(86) International application number: PCT/JP2010/073771
(87) International publication number: WO 2011/129034

(57) **Abstract**

An object of the present invention is to provide an easy-to-handle, before and after dilution with water, concentrated liquid cleanser composition and a production method thereof.

A concentrated cleanser composition of the present invention is characterized by comprising: (A) an anionic surfactant, (B) an amphoteric surfactant, (C) 5 to 15 mass % of a monohydric or dihydric alcohol, (D) 8 to 18 mass % of a nonionic surfactant with the IOB value of 0.8 to 1.1 and the molecular weight of 500 or lower, and (E) 45 mass % or less of water, wherein the sum of (A) and (B) is 40 to 60 mass %; wherein the blending ratio (C):(D) is 3.5:1 to 1:2.5; and wherein the viscosity at 30 °C is 300 mPa • s or higher when the composition is diluted until the concentration of (A) and (B) becomes 15 mass %.

## Description

### RELATED APPLICATIONS

This application claims the priority of Japanese Patent Application No. 2010-091799 filed on April 12, 2010, which are incorporated herein by reference.

### FIELD OF THE INVENTION

The present invention relates to a concentrated liquid cleanser composition, in particular, relates to a concentrated liquid cleanser composition comprising a high concentration of an ionic surfactant and suitable for use by dilution with water and also relates to a production method thereof.

### BACKGROUND OF THE INVENTION

As cleansers wherein ionic surfactants are used, hair or skin cleanser compositions can be listed. Among hair or skin cleanser compositions, there are solid compositions such as bar soap and a syndet bar and creamy compositions such as facial cleansing foam besides liquid compositions. The main component in solid or creamy cleansers is an anionic surfactant in which the hydrophilic part is a carboxylic acid. However, the feeling in use when applied on the hair or skin was not good, or foaming/cleansing power with hard water was not good. Solid or creamy cleansers wherein the main component is an anionic surfactant in which the hydrophilic part is not a carboxylic acid has been known; however, the feeling in use and the ease of use have not been good. Thus, from the standpoint of the ease of use, most formulations for hair or skin cleanser compositions are liquid. Such liquid cleanser compositions generally comprise 10 to 20% of an ionic surfactant and 60 to 80% of water.

On the other hand, in recent years, the production volume and distribution volume of hair or skin cleanser compositions account for an overwhelming percentage of the products in the cosmetic field. In the conventional liquid cleanser compositions having heavy weight and bulkiness, because of large water content (60 to 80%), the reduction of energy for the distribution has been a problem. As an approach to such a problem, efforts have been made for the improvement of transport technology and means (efficiency improvement in logistics, modal shift, technology improvement in product packaging, etc.) and for the saving of container weight (reduction of container weight, sale of refill products, etc.).

As the means for energy reduction, in addition to the above-described effort in the distribution system, the energy suppression in the production of liquid cleanser compositions can be considered. For the high-water-content liquid cleanser compositions, however, highly energy-consuming heating and cooling processes are necessary. Thus, there is limitation in energy reduction from the standpoint of production, and a satisfactory effect has not been achieved.

For liquid hair or skin cleanser compositions such as hair shampoo, the above-described technologies have been applied to achieve energy reduction. However, as to the reduction of water itself, which is a major cause of energy consumption, in the composition, sufficient efforts have not been made because of the earlier described problems such as the feeling in use etc.

Also from the viewpoint of users, the demand for light-weight and compact hair shampoo, which can be used outdoors, is increasing year after year.

Thus, the reduction of water content in hair shampoo accords with the reduction of a large amount of energy in production and transport, and it accords with the expectation to contribute to the improvement of the global environment and with the market demand.

One of the main reasons that a large amount of water is contained in liquid cleanser compositions is that the ionic surfactant raw material is widely distributed in the form of 25 to 35% aqueous solution of the surfactant.

Accordingly, in order to decrease the water content in the cleanser composition, it is necessary to increase the concentration of a surfactant aqueous solution. However, if the surfactant concentration becomes high, the viscosity of the solution normally increases significantly; thus the product preparation becomes very difficult. Some of anionic surfactants are distributed in the form of 65 to 75% aqueous solution. However, when mixed with other water-containing components and water, a significant viscosity increase takes place. Thus, special mixing equipment was necessary for its use. Therefore, in order to allow a surfactant to be easily usable in a state of high concentration, the following technologies have been developed.

In the publication of Japanese Patent No. 3644658, it is disclosed that the foaming property and moisture-retaining property can be provided by blending both an ethylene glycol long-chain ester and a specific polyhydric alcohol in a hair cleanser wherein the blending quantity of the surfactants selected from anionic, amphoteric, and nonionic surfactants is large (30 mass % or higher).

However, the technology in the publication of Japanese Patent No. 3644658 is not intended to decrease the water content in the composition. Thus, there is no reference to the gelation of the composition, when the amount of water is decreased, due to a polyhydric alcohol or to its suppression.

On the other hand, in Japanese Unexamined Patent Application No. 2006-265547 and Japanese Unexamined Patent Application No. 2007-55997, surfactant compositions wherein a high concentration of polyoxyethylene alkyl ether sulfate salt or alkyl sulfate salt is easily usable are disclosed. In the compositions, viscosity increase and gelation problems, under the conditions of high surfactant concentration, are solved by blending combined water-soluble salts to glyceryl ether or diglyceryl ether having an alkyl group or an alkenyl group (Japanese Unexamined Patent Application No. 2006-2655472) or to an alkylene oxide adduct of an alcohol with a specific structure (Japanese Unexamined Patent Application No. 2007-55997).

In Japanese Unexamined Patent Application Publication (Translation of PCT Application) No. 2009-536250, a compact liquid laundry detergent composition comprising 5 to 45 weight % of water and non-aminofunctional solvent in total is described. The composition has, when measured at 20 s⁻¹, a neat viscosity Vn of 1 Pa ·s to 10 Pa ·s and a diluted viscosity Vd of 0.5 Vn or less, and it becomes preferably smaller by dilution. That is, the composition described in Japanese Unexamined Patent Application Publication (Translation of PCT Application) No. 2009-536250 is a laundry detergent composition, wherein a drastic viscosity decrease takes place before and after dilution.

### DISCLOSURE OF THE INVENTION

### PROBLEM TO BE SOLVED BY THE INVENTION

As shown in the technology of the above-described each patent literature, when a highly concentrated surfactant is handled, a component that corresponds to a gelation inhibitor has been blended to improve handling and facilitate the dilution with water.
It is possible to achieve easy-to-handle viscosity, by blending such a gelation inhibitor, for a hair cleanser composition containing a high concentration of a surfactant, namely, with reduced water content. However, it is not desirable to use a highly-concentrated surfactant, as it is, from the standpoint of skin irritation/safety and because of the burden/difficulty in that the entire hair should be washed with a small amount of cleanser. Thus, it is considered to be preferable to dilute, in view of the safety and usability of a hair cleanser composition, a highly-concentrated surfactant composition with water, during use or before use after transport and delivery, and use in a liquid state of normal concentration.

On the other hand, in a composition wherein the viscosity increase due to a highly concentrated surfactant is suppressed by a gelation inhibitor, the viscosity obviously decreases with an increase of dilution with water. Thus, a texture in use of a moderately viscous normal liquid cleanser composition cannot be obtained. In the publication of Japanese Patent No. 3644658, Japanese Unexamined Patent Application No. 2006-265547 and Japanese Unexamined Patent Application No. 2007-55997 described above, the viscosity during dilution and that after dilution of the composition were not investigated. In the technology of Japanese Unexamined Patent Application Publication (Translation of PCT Application) No. 2009-536250, it is described that the viscosity of the composition becomes half or less by dilution. Thus, a concentrated cleanser composition that displays usable viscosity after being diluted with water to a normal concentration has not been realized as a product or half-finished product/raw material.

The present invention was made in view of the above-described problems, and an object is to provide an easy-to-handle, before and after dilution with water, concentrated liquid cleanser composition and a production method thereof.

### MEANS TO SOLVE THE PROBLEM

The present inventors have diligently studied to solve the above-described problems. As a result, the present inventors have found that a composition wherein a monohydric or dihydric alcohol and a specific nonionic surfactant are blended as the media of an anionic surfactant and an amphoteric surfactant, which are cleansing components, has a small viscosity change by water dilution, the dilution is easy, and the composition has easy-to-handle viscosity before and after dilution, thus leading to the completion of the present invention.

That is, the concentrated cleanser composition of the present invention is characterized by comprising: (A) an anionic surfactant, (B) an amphoteric surfactant, (C) 5 to 15 mass % of a monohydric or dihydric alcohol, (D) 8 to 18 mass % of a nonionic surfactant with the IOB value of 0.8 to 1.1 and the molecular weight of 500 or lower, and (E) 45 mass % or less of water, wherein the sum of (A) and (B) is 40 to 60 mass %; wherein the blending ratio (C):(D) is 3.5:1 to 1:2.5; and wherein the viscosity at 30 °C is 300 mPa • s or higher when the above-described composition is diluted until the concentration of (A) and (B) becomes 15 mass %.

In addition, in the above-described concentrated liquid cleanser composition, it is preferable that (D) the nonionic surfactant is a long-chain fatty acid N-methylethanolamide with the average number of carbon atoms of 10 to 14 and/or a diethylene glycol long-chain fatty acid ester with the average number of carbon atoms of 10 to 14.

In addition, in the above-described concentrated liquid cleanser composition, it is preferable that (A) the anionic surfactant comprises a polyoxyethylene alkyl ether sulfate salt.

In addition, in the above-described concentrated liquid cleanser composition, it is preferable to further comprise an organic or inorganic salt is also contained.

In addition, the method of use of the above-described concentrated liquid cleanser composition of the present invention is characterized by mixing the composition with water.

In addition, the production method of the above-described concentrated liquid cleanser composition of the present invention is characterized by comprising: mixing (C) the monohydric or dihydric alcohol and (D) the nonionic surfactant into an aqueous solution of (A) the anionic surfactant and; and adding and mixing an aqueous solution of (B) the amphoteric surfactant into the mixture.

In addition, the production method of the liquid cleanser composition of the present invention is characterized by mixing the concentrated liquid cleanser composition with water.

### EFFECT OF THE INVENTION

According to the present invention, a concentrated-type liquid cleanser composition, which is usable by easy dilution with water while maintaining appropriate viscosity, can be obtained. The composition can allow the energy reduction during production and during transportation without losing its quality as a liquid cleanser. In addition, it can allow the reduction of energy necessary for the use and disposal of containers and outer packagings; therefore, a contribution to the improvement of the global environment can be expected. In addition, the concentrated liquid cleanser composition of the present invention has low water content and it is compact. Thus, it can be used in an easy-to-handle form and it is very favorable for carrying on to airplanes and outdoor usage.

In addition, the concentrated cleanser composition of the present invention does not contain a large amount of water. Therefore, the production in a short time and with low energy is more possible than ever before.

### BRIEF DESCRIPTION OF THE DRAWINGS

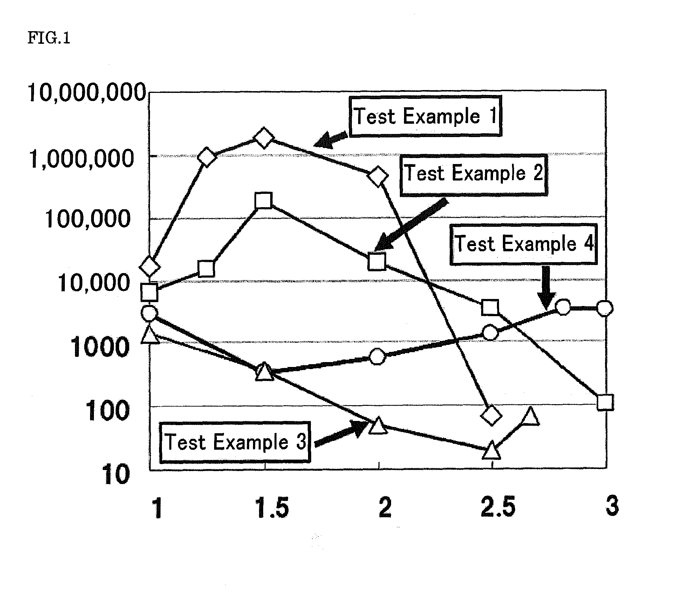

Fig. 1 is a graph which shows the variation of composition viscosity according to dilution rate of a cleansing component.

### DESCRIPTION OF THE PREFERRED EMBODIMENT

The present invention is a concentrated liquid cleanser composition comprising, as the components, (A) an anionic surfactant, (B) an amphoteric surfactant, (C) a monohydric or dihydric alcohol, (D) a nonionic surfactant with the IOB value of 0.8 to 1.1 and the molecular weight of 500 or lower, and (E) water. The composition of the present invention can be easily diluted to a desired concentration, while the appropriate viscosity is being maintained, because the viscosity change is very small during the dilution with water to a specific concentration of (A) + (B) components.

In the conventional liquid cleanser production, it is not impossible to produce a liquid cleanser composition containing concentrated cleansing components by raising the concentration by blending large amounts of anionic and amphoteric surfactants as cleansing components, or by lowering the water content of the liquid cleanser by means such as drying. However, when such a composition is diluted with water, a region where the viscosity increases enormously appears during the process. For example, Test Example 1 in Fig. 1 shows the variation of composition viscosity (30 °C) when 70% aqueous solution of sodium POE lauryl ether sulfonate (hereinafter referred to as LES) (Texapon N70, manufactured by Cognis Corporation), which is a general cleansing component, is diluted at various dilution rates.

According to Fig. 1, the viscosity of the above-described 70% LES aqueous solution rapidly increases from the start of dilution, and the peak (about 1 million mPa • s) is reached when the dilution rate is 1.5 times. When the dilution rate exceeds 2 times, the viscosity rapidly decreases. When the dilution rate is 2.5 times, the viscosity falls to about 100 mPa • s. That is, when a concentrated liquid cleanser in which an anionic surfactant, as the cleansing component, is blended 3 times the normal concentration is diluted, at first it becomes hard to the extent that the mixing becomes difficult; subsequently it becomes rapidly loose, showing an unstable behavior.

Test Example 2 in Fig. 1 shows the viscosity change, by the dilution, of the mixing base (Plantapon 611 C, manufactured by Cognis Corporation), of the cleanser which comprises sodium POE lauryl ether sulfate, coconut oil fatty acid amidopropyl betaine solution, and alkyl (8-16) glucoside, and in which the concentration of the anionic surfactant is about 45% and the total amount of surfactants is about 64%. Similarly to Test Example 1, the viscosity of the composition of Test Example 2 rapidly increases/decreases according the dilution rate. Thus, the composition is very difficult to handle as a concentrate for dilution-use.

Such a viscosity change by the dilution of the cleansing component is considered to be due to the change in the aggregate structure of the active agent.

Generally, if a surfactant exceeds the critical micelle concentration (hereinafter referred to as "cmc") in solvent (water), string micelles are formed. If the concentration is further increased, the aggregate structure is known to change to a lamellar liquid crystal structure via a hexagonal liquid crystal structure. Accordingly, when a highly concentrated surfactant is diluted only with water, the aggregate structure changes from lamellar liquid crystal → hexagonal liquid crystal → string micelle. Among them, lamellar liquid crystals and hexagonal liquid crystals take a highly viscous gel-like structure. In particular, hexagonal liquid crystals are very hard gel. That is, the high-viscosity region shown by Test Examples 1 and 2 in Fig. 1, is considered to be the region wherein the surfactant aggregate structure changes to a lamellar liquid crystal structure or to a hexagonal liquid crystal structure.

On the other hand, when a high-solubility alcohol is added as the solvent of a cleansing component, the cmc increases and the surfactant maintains a string micelle structure even at a high concentration state. Test Example 3 in Fig. 1 is the viscosity change of the system wherein 8 mass % of dipropylene glycol is blended to the mixing base of a cleanser comprising a net amount of 32 mass % of sodium POE lauryl ether sulfate and a net amount of 8 mass % of an amphoteric surfactant. As shown in Test Example 3, because the surfactant maintains string micelles, the viscosity increase due to the change to lamellar liquid crystals and hexagonal liquid crystals is not observed. However, the viscosity decreases immediately when the dilution rate increases, and the viscosity suitable for cleansing (about 300 mPa • s) cannot be maintained. Thus, it becomes difficult to use as a cleanser composition. Such a decrease in viscosity is considered to take place because an alcohol weakens the packing state of hydrophilic groups in the micelle and increases fluidity.

In the present invention, a nonionic surfactant is also used as a cosurfactant. The cosurfactant gradually penetrates into aggregates and produces a strengthening effect of hydrophobic interaction while an alcohol weakens the packing of hydrophilic groups. Accordingly, as displayed by Test Example 4, wherein the nonionic surfactant, coconut oil fatty acid N-methylethanolamide (Aminon C11, manufactured by Kao Corporation) is blended into Test Example 3 in Fig. 1, the fluidity of string micelles does not increase by dilution with water and moderate viscosity can be maintained continuously.

At first, components (A) to (E) that are blended in the present invention will be explained.

### (A) Anionic surfactant

As the anionic surfactant that is blended in the present invention, those normally used in cosmetics, pharmaceuticals, etc. can be used. Examples of anionic surfactants suitable for the present invention include polyoxyethylene alkyl ether sulfate salts represented by the below-described general formula (I).

R-O-(CH₂CH₂O)ₙ-SO₃X (I)

In the above-described general formula (I), R represents a linear or branched chain alkyl group, and the number of carbon atoms is preferably 10 to 16, and more preferably 12 to 14. In addition, n represents an integer from 1 to 3. Examples of X include, in addition to a hydrogen atom, an alkali metal, an alkaline earth metal, an ammonium ion, a lower alkanolamine cation, a lower alkylamine cation, and a basic amino acid cation.

Examples of the above-described polyoxyethylene alkyl ether sulfate salts include sodium POE(1-3) alkyl ether sulfate, triethanolamine POE(1-3) alkyl ether sulfate, ammonium POE(1-3) lauryl ether sulfate, sodium POE(1-3) lauryl ether sulfate, etc.

As other anionic surfactants suitable for the present invention, alkyl sulfate salts represented by the below-described general formula (II) can be listed.

R-O-SO₃X (II)

In the above-described general formula (II), R represents a linear or branched chain alkyl group, and the number of carbon atoms is preferably 10 to 16, and more preferably 12 to 14. Examples of X include, in addition to a hydrogen atom, an alkali metal, an alkaline earth metal, an ammonium ion, a lower alkanolamine cation, a lower alkylamine cation, and a basic amino acid cation.

Examples of the above-described alkyl sulfate salts include ammonium lauryl sulfate, potassium myristyl sulfate, sodium lauryl sulfate, triethanolamine cocoyl sulfate, etc.

In addition, as other anionic surfactants suitable for the present invention, N-acyltaurine salts represented by the below-described general formula (III) can be listed.

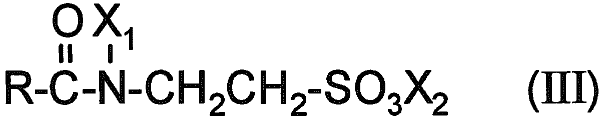

In the above-described general formula (III), R represents a linear or branched alkyl group, the number of carbon atoms is preferably 10 to 16, and more preferably 12 to 14. X₁ represents a hydrogen atom or a methyl group. Examples of X₂ include, in addition to a hydrogen atom, an alkali metal, an alkaline earth metal, an ammonium ion, a lower alkanolamine cation, a lower alkylamine cation, and a basic amino acid cation.

Examples of the above-described N-acyl taurine salts include sodium N-cocoyl methyl taurate, sodium N-lauroyl methyl taurate, sodium N-myristoyl methyl taurate, sodium
N-stearoyl methyl taurate, sodium coconut oil fatty acid methyl taurate, etc.

In addition, as other anionic surfactants suitable for the present invention, N-acylamino acid salts represented by the below-described general formulas (IV) and (V) can be listed.

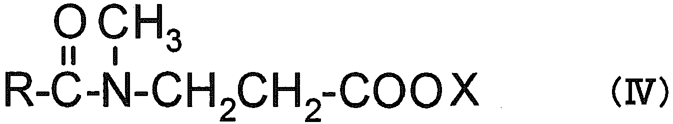

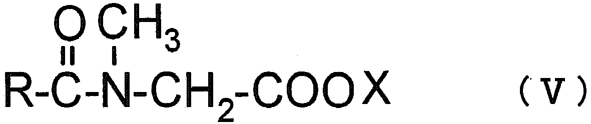

In the above-described general formula (IV) and (V), R represents a linear or branched alkyl group, the number of carbon atoms is preferably 10 to 16, and more preferably 12 to 14. Examples of X include, in addition to a hydrogen atom, an alkali metal, an alkaline earth metal, an ammonium ion, a lower alkanolamine cation, a lower alkylamine cation, and a basic amino acid cation.

Examples of the above-described N-acylamino acid salts include sodium lauroyl methyl alanine, sodium coconut oil fatty acid sarcosinate, triethanolamine coconut oil fatty acid sarcosinate, sodium lauroyl sarcosinate, potassium lauroyl sarcosinate, etc.

In addition, as other anionic surfactants suitable for the present invention, hydroxy ether carboxylate salts represented by the below-described general formula (VI) can be listed.

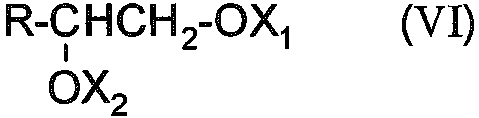

In the above-described general formula (VI), R represents a linear or branched alkyl group or alkenyl group, and the number of carbon atoms is preferably 4 to 34, and more preferably 8 to 25. If the number of carbon atoms of an alkyl group or an alkenyl group is less than 4 or exceeds 34, a satisfactory foaming property and a satisfactory feeling in use may not be obtained.

At least one of X₁ and X₂ is -CH₂COOM or -CH₂CH₂COOM, and the other can be a hydrogen atom. M is a hydrogen atom, an alkali metal, an alkaline earth metal, an ammonium ion, a lower alkanolamine cation, a lower alkylamine cation, or a basic amino acid cation.

In addition, as other anionic surfactants suitable for the present invention, polyoxyethylene alkyl ether carboxylate salts represented by the below-described general formula (VII) can be listed.

R-O-(CH₂CH₂O)nCH₂COOM (VII)

In the above-described general formula (VII), R represents a linear or branched alkyl group or alkenyl group, and the number of carbon atoms is preferably 4 to 34, and more preferably 8 to 25. In addition, n is 0 or an integer 1 or higher. M represents a salt-forming cation such as an alkali metal, an alkaline earth metal, an ammonium, an alkanolamine, etc.

Among the above-described anionic surfactants, in the present invention, polyoxyethylene alkyl ether sulfate salts or polyoxyethylene alkyl ether carboxylate salts are preferable, polyoxyethylene alkyl ether sulfate salts are more preferable, and sodium POE(2) lauryl ether sulfate is most preferable. As the above-described material, the commercial products, for example, Texapon N70 (manufactured by Cognis Corporation), Sinolin SPE-1250 (manufactured by New Japan Chemical Co., Ltd), etc. can be used.

In the concentrated cleanser composition of the present invention, the blending quantity of (A) the anionic surfactant is a net amount of 20 to 40% of the composition, preferably 25 to 30 mass %, and more preferably 26 to 28 mass %. If the blending quantity of component (A) is less than a net amount of 20 mass % of the composition or exceeds 40 mass %, the pre-dilution viscosity of the composition becomes high and the preparation may become difficult, the viscosity of the composition during dilution significantly increases and the dilution may become difficult, or the post-dilution viscosity of the composition becomes significantly low and the handling may become difficult.

The above-described commercial anionic-surfactant raw materials are being supplied to the market as a flowable aqueous solution with a concentration of 25 to 35 % or 65 to 75% or a waterless cake with a concentration of 85 to 100%. Normally, a raw material aqueous solution with a concentration of 25 to 35% is used. In the present invention, from the viewpoint of the acquisition of a concentrated composition, it is preferable to use a raw material with a higher concentration of anionic surfactant, and preferably that with a concentration of 50% or higher. It is also preferable, from the viewpoint of the ease of production, to use a raw material dissolved in a liquid instead of a dried and powdered raw material as the anionic surfactant. The higher the net concentration of active agent in the above-described raw material, a concentrated cleanser composition containing the denser anionic surfactant can be obtained.

### (B) Amphoteric surfactant

For the amphoteric surfactant that is blended in the present invention, those normally used in cosmetics, pharmaceuticals, etc. can also be used. As amphoteric surfactants suitable for the present invention, for example, acetic acid betaine type amphoteric surfactants represented by the below-described general formula (VIII) and (IX) can be listed.

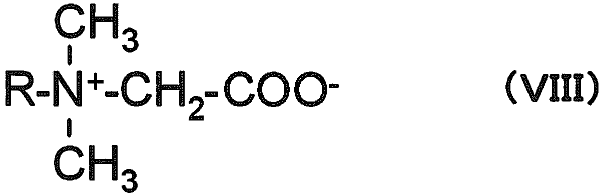

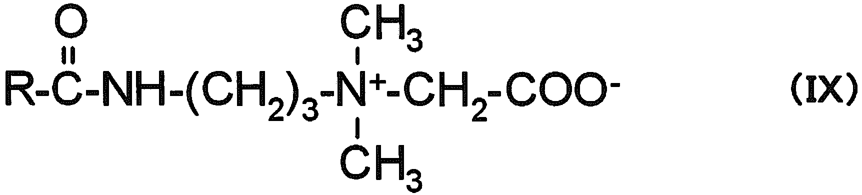

In the above-described general formula (VIII) and (IX), R represents a linear or branched alkyl group, and the number of carbon atoms is preferably 10 to 16, and more preferably 12 to 14.

Examples of the above-described acetic acid betaine type amphoteric surfactants include lauryldimethylamino acetic acid betaine, coconut oil fatty acid amidopropyldimethylamino acetic acid betaine, palm kernel oil amidopropyldimethylamino acetic acid betaine, etc.

In addition, as other amphoteric surfactants suitable for the present invention, imidazoline-type amphoteric surfactants represented by the below-described general formula (X) can be listed.

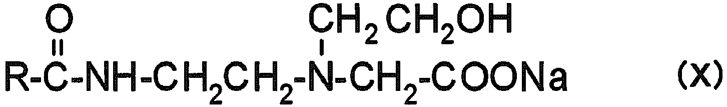

In the above-described general formula (X), R represents a linear or branched alkyl group, the number of carbon atoms is preferably 10 to 16, and more preferably 12 to 14.

Examples of the above-described imidazoline-type amphoteric surfactants include sodium N-coconut oil fatty acid acyl-N-carboxymethyl-N-hydroxyethyl ethylenediamine, sodium 2-undecyl-N,N-(hydroxyethylcarboxymethyl)-2-imidazoline, etc.

In addition, as other amphoteric surfactants suitable for the present invention, tertiary amine oxides represented by the below-described general formula (XI) can be listed.

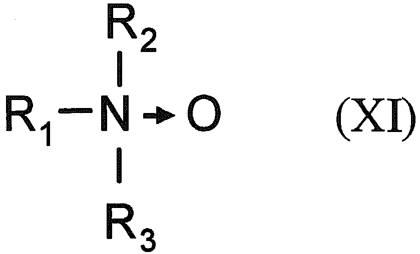

In the above-described general formula (XI), R₁ represents a linear or branched alkyl group or alkenyl group having 8 to 22 carbon atoms, and R₂ and R₃ represent a methyl group or an ethyl group, respectively.

Examples of the above-described tertiary amine oxides include coconut oil fatty acid dimethylamine oxide, lauric acid dimethylamine oxide, tetradecyldimethylamine oxide, dodecyldimethylamine oxide, etc.

Among the above-described amphoteric active agents, especially preferable amphoteric surfactants in the present invention are lauryldimethylamino acetic acid betaine, coconut oil fatty acid amidopropyldimethylamino acetic acid betaine, palm kernel oil amidopropyldimethylamino acetic acid betaine, and sodium 2-undecyl-N,N,N-(hydroxyethylcarboxymethyl)-2-imidazoline. As the above-described materials, for example, commercial products such as Anon BL-SF (manufactured by NOF Corporation), Lebon 2000 SF (manufactured by Sanyo Chemical Industries, Ltd.), Genagen Cab 818J (manufactured by Clariant Japan Co., Ltd.), Tego Betain C60 (manufactured by Degussa Corporation), Dehyton PK 45 (manufactured by Cognis Corporation), Nissananon BDC-SF (manufactured by NOF Corporation), Obazolin 662N (manufactured by Toho Chemical Industry Co., Ltd.), etc. can be used.

The blending quantity of (B) the amphoteric surfactant in the present invention is a net amount of 10 to 20 mass % of the composition, preferably 11 to 17 mass %, and more preferably 14 to 16 mass %. If the blending quantity of component (B) is less than 10 mass % of the composition, the post-dilution viscosity of the solution may become significantly low. If the blending quantity exceeds 20 mass %, the pre-dilution viscosity of the composition becomes high and the preparation becomes difficult, or the viscosity of the composition significantly increases during dilution and the dilution may become difficult.

The above-described commercial amphoteric surfactant raw materials are normally supplied to the market as 25 to 40% aqueous solutions. In the present invention, from the viewpoint of the acquisition of concentrated compositions, it is preferable to use a raw material of amphoteric surfactant with a higher concentration, preferably 35% or higher. The higher the net concentration of an active agent in the above-described raw material, the cleanser composition containing a denser amphoteric surfactant can be obtained.

In the present invention, the total blending quantity of the above-described component (A) and component (B), which are cleansing components, is a net amount of 40 to 60 mass % of the composition. If the total blending quantity of these surfactants is less than a net amount of 40 mass %, the merit as the concentrated composition is reduced and it is not attractive. On the other hand, if the total blending quantity of these surfactants exceeds 60 mass %, a composition that is easily dilutable with water cannot be obtained.

### (C) Monohydric or dihydric alcohol

The concentrated cleanser composition of the present invention comprises, as the medium of the above-described (A) and (B) components, one or more monohydric or dihydric alcohol and one or more nonionic surfactants.

The monohydric or dihydric alcohol that is blended in the present invention is not limited in particular. However, when we consider the storage stability of concentrated cleanser compositions and the ease of handling at the time of dilution, it is preferably a liquid at the temperature lower than 50 °C wherein the storage/use of the composition is assumed.

Examples of these include, as monohydric alcohol, ethanol, isostearyl alcohol and jojoba alcohol; as dihydric alcohol, dipropylene glycol, 1,3-butylene glycol, propylene glycol, 1,2-pentanediol, isoprene glycol, hexylene glycol and 1,2-octanediol. In the present invention, ethanol, dipropylene glycol, butylene glycol, propylene glycol and isoprene glycol are particularly preferable.

The blending quantity of (C) the monohydric or dihydric alcohol in the present invention is 5 to 15 mass % of the composition, and more preferably 10 to 13 mass %. If the blending quantity of component (C) is less than 5 mass % of the composition, the pre-dilution viscosity of the composition becomes high and the preparation becomes difficult, or the viscosity of the composition significantly increases during dilution and the dilution may become difficult. If the blending quantity exceeds 15 mass %, the post-dilution viscosity of the solution becomes significantly low and the handling may become difficult.

### (D) Nonionic surfactant with the IOB value of 0.8 to 1.1 and the molecular weight of 500 or lower

The nonionic surfactant used in the concentrated cleanser composition of the present invention is a compound in which the IOB value on the organic conceptual diagram is 0.8 to 1.1 and the molecular weight is 500 or lower.

Examples of such nonionic surfactants include those that satisfy the above-described IOB range and the above-described molecular weight range among diethylene glycol long-chain fatty acid ester, propylene glycol long-chain fatty acid ester, long-chain fatty acid diethanolamide, long-chain fatty acid N-methylethanolamide, long-chain fatty acid (POE) 2 monoethanolamide, etc. If the IOB value is lower than 0.8 or the IOB value is higher than 1.1, problems are generated in that a significant viscosity increase takes place during dilution, the post-dilution viscosity becomes significantly low, or the solution composition after dilution is not one phase anymore. If the molecular weight exceeds 500, the post-dilution viscosity of the composition becomes significantly low. In the present invention, a diethylene glycol fatty acid ester with the average number of carbon atoms of 10 to 14 or a fatty acid N-methylethanolamide with the average number of carbon atoms of 10 to 14 is especially preferable.

As commercial nonionic surfactants, for example, Genapol DEL (manufactured by Clariant Japan Co., Ltd.), as diethylene glycol laurate, and Aminon C-11(manufactured by Kao Corporation), as coconut oil fatty acid N-methylethanolamide, can be suitably used.

The blending quantity of (D) the nonionic surfactant in the present invention is 8 to 18 mass % of the composition, and more preferably 10 to 15 mass %. If the blending quantity of component (D) is less than 8 mass % of the composition, the post-dilution viscosity of the solution may become significantly low. If the blending quantity exceeds 18 mass %, the pre-dilution viscosity of the composition becomes high and the preparation becomes difficult, or the viscosity of the composition significantly increases during dilution and the dilution may become difficult.

In the present invention, the total blending quantity of the above-described component (C) and component (D) is preferably 20 to 30 mass %, and more preferably 22 to 24 mass % of the composition containing a net amount of 40 mass % or more of component (A) and component (B). In particular, the blending ratio of (C) and (D) is (C):(D) = 3.5:1 to 1:2.5 and preferably 2:1 to 1:1.5. If the total blending quantity is less than 20 mass % , that is, the percentage of component (A) and component (B) is too large, the dilution of the concentrated cleanser composition with water may be difficult. If the total blending quantity exceeds 30 mass %, that is, the percentage of component (A) and component (B) is too small, the dilution of the concentrated cleanser composition into water is easy; however, the post-dilution viscosity of the solution may become too low.

### (E) Water

The concentrated cleanser composition of the present invention contains 45 mass % or less of water. In the present invention, water can be suitably added as a single component; however, the blending of water contained as the solvent for surfactant raw materials is normally satisfactory. Generally, anionic surfactants and amphoteric surfactants are commercially available as about 25 to 40% high-concentration aqueous solutions. Accordingly, by using these surfactants in the state of aqueous solutions, the blending of water in the present invention can be achieved.

The presence of water exceeding 45 mass % is not preferable from the viewpoint of the reduction of energy, which is used for the production and transport of products.

The concentrated cleanser composition of the present invention can be easily produced by mixing (C) a monohydric or dihydric alcohol and (D) a nonionic surfactant into an aqueous solution of (A) an anionic surfactant and then adding and mixing an aqueous solution of (B) an amphoteric surfactant without encountering a high-viscosity region, which is generated when water is reduced from the composition.

Specifically, for example, a monohydric or dihydric alcohol and an aqueous solution of a nonionic surfactant are added into an aqueous solution of an anionic surfactant at room temperature, and it is stirred until the solution becomes uniform. On this occasion, heating may be applied if an increase in viscosity takes place and the bubble entrainment tends to take place because of mixing. Then, an aqueous solution of an amphoteric surfactant is added and stirred; thus the composition of the present invention can be obtained.

When the conventional liquid cleanser composition with high water content is produced, the mixing order of components has no significant effect on the production of the composition. However, in the production of the concentrated cleanser composition of the present invention, if the addition order is different from the above-described order, the viscosity may significantly increase and the production of the composition may become difficult.

The concentrated cleanser composition of the present invention consisting of such components and by such a production method can be used, for example, as a hair shampoo precursor composition before applying on the hair as hair shampoo. That is, the concentrated cleanser composition of the present invention can be used in the same way as the conventional hair shampoo by mixing and diluting its suitable amount with water at the time of use. The specific dilution rate, in the present invention, can be suitably adjusted by the blending quantities of the above-described essential components and their blending ratios. Normally, the cleansing effectiveness comparable to that of the conventional liquid hair shampoo can be obtained by diluting until the total concentration of the above-described component (A) and component (B) becomes a net amount of 15 mass %. Accordingly, if the blending quantity of (A) + (B) is a net amount of 40 mass % of the composition, for example, the suitable dilution rate is about 2.6.

In this patent application, the above-described composition was diluted with water until the sum of (A) and (B) concentrations became a net amount of 15 mass %, and the range of viscosity change during dilution and the post-dilution composition viscosity were evaluated. However, 15 mass % was only used as the standard rate of dilution, and the dilution rate of the concentrated cleanser composition of the present invention is not limited by this rate.

The higher the temperature of water used for dilution, the faster the speed of dilution. However, the dilution can be satisfactorily achieved even with water at room temperature (20 to 30 °C). The hardness of water used for dilution hardly affects the speed of dilution, and the dilution can be satisfactorily carried out, depending upon the dilution method used, even when the hardness is high.

The concentrated liquid cleanser composition of the present invention may be used by diluting a necessary amount on the palm at each usage or by diluting the bulk material in a suitable size of container in advance of use.

The concentrated liquid cleanser composition of the present invention has no high-viscosity region that is dependent on the water content. Therefore, an easy-to-handle viscosity can be maintained before dilution-during dilution-after dilution.

Generally, it is considered that a liquid composition can be easily mixed if the viscosity at 30 °C is 20000 mPa • s or lower, and the mixing becomes difficult if the viscosity exceeds 50000 mPa • s. Accordingly, the above-described liquid viscosity of the composition of the present invention (original solution) and the liquid viscosity during the dilution of the composition with water until the total concentration of component (A) and component (B) becomes a net amount of 15 mass % is 50000 spa • s or lower at 30 °C and at ordinary pressure, and preferably 20000 mPa • s or lower.

In addition, the post-dilution liquid viscosity of the concentrated liquid cleanser composition of the present invention is arranged to be 300 mPa • s or higher at 30 °C and at ordinary pressure. If the post-dilution viscosity of the composition, namely, the viscosity at the time of use is lower than 300 mPa • s, the viscosity is too low to handle and it is difficult to apply the composition on the entire hair. Especially, it is preferable to adjust the post-dilution liquid viscosity to be 300 to 20000 mPa • s s (30 °C) by considering the ease of cleanser handling at the time of use. If the viscosity at the time of use exceeds 20000 mPa • s, the viscosity is too high and the application to a cleansing target and the transfer from or into the container become difficult.

That is, in this patent application, a composition that is "easy-to-handle before and after dilution" means the composition wherein the above-described viscosity increase (viscosity change), during dilution, due to the effect of a surfactant aggregate structure is up to 50000 mPa • s or lower and preferably 20000 mPa • s or lower, and the viscosity after dilution (at the time of use) is 300 mPa • s or higher and preferably 300 to 20000 mPa • s.

In addition, it is preferable that the phase state of the composition of the present invention and the phase state of its diluted material is one phase. If it is separated into two phases, it is undesirable in the ease of composition handling and also in its stability.

In addition, it is preferable to blend an organic or inorganic salt in the concentrated liquid cleanser composition of the present invention in order to lower the viscosity of the composition in the high-viscosity region and facilitate the dilution into water.

Examples of salts include organic salt, amino-acid salt and inorganic salt. Examples of organic salts include hydrochloride salt, metallic salt (sodium salt and potassium salt) and amine salt of citric acid, lactic acid, oxalic acid, succinic acid, malic acid, acidum tartaricum and sulfonic acid. Examples of amino-acid salts include hydrochloride salt, metallic salt (sodium salt and potassium salt) and amine salt of glycine, alanine, proline, lysine, asparagine acid and glutamic acid. Examples of inorganic salts include carbonate, phosphate, nitrate, borate, sulfate, sulfite and halide of sodium, potassium, magnesium, calcium and ammonium (e.g. sodium chloride, potassium chloride and ammonium chloride).

In the present invention, the blending quantity of a salt is preferably 0.1 to 5.0 mass % of the composition, and more preferably 1.0 to 2.0 mass %, and it is preferable to blend sodium chloride, ammonium chloride, or sodium citrate.

In addition to the above components, other components which are usually used in cosmetics and medicines may be incorporated into a concentrated cleanser composition of the present invention within the range not impairing the effect of the invention.

Examples of other components include oil component, cationic surfactant, powder component, natural polymer, synthetic polymer, thickener, ultraviolet absorber, sequestrant, pH adjuster, skin nutritional supplement, vitamin, antioxidant, auxiliary antioxidant and perfume.

Examples of oil components include liquid oil, solid oil, hydrocarbon oil and silicone oil.

Examples of liquid oils include avocado oil, camellia oil, turtle oil, macadamia nut oil, corn oil, mink oil, olive oil, rapeseed oil, egg oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, paulownia oil, Japanese tung oil, jojoba oil, germ oil and triglycerin.

Examples of solid oils include cacao butter, coconut oil, horse fat, hardened coconut oil, palm oil, beef tallow, mutton tallow, hardened beef tallow, palm kernel oil, lard, beef bone tallow, Japan wax kernel oil, hardened oil, neats-foot oil, Japan wax, hardened castor oil.

Examples of hydrocarbon oils include liquid paraffin, ozocerite, squalane, pristine, paraffin, ceresin, squalene, vaseline and microcrystalline wax.

Examples of silicone oils include linear polysiloxane (e.g. dimethylpolysiloxane, methylphenyl polysiloxane and diphenylpolysiloxane); cyclic polysiloxane (e.g. octamethylcyclotetrasiloxane, decamethylcyclopentasiloxane and dodecamethylcyclohexasiloxane); silicone resin forming three-dimensional network structure; silicone rubber; various kinds of modified polysiloxane (e.g. amino modified polysiloxane, polyether modified polysiloxane, alkyl modified polysiloxane, polyether/alkyl co-modified polysiloxane, fluorine modified polysiloxane, polyoxyethylene/polyoxypropylene co-modified polysiloxane, linear aminopolyether modified polysiloxane, amidoalkyl modified polysiloxane, aminoglycol modified polysiloxane, aminophenyl polysiloxane, carbinol modified polysiloxane, polyglycerin modified polysiloxane and polyglycerin/alkyl co-modified polysiloxane); dimethiconol; and acrylic silicone.

As the blending conditions, the silicone oil may be solubilized or emulsified in the composition. When it is emulsified, the particle size is the same as that in the technology of normal cleanser compositions.

Examples of cationic surfactants include alkyltrimethylammonium salt (e.g. stearyltrimethylammonium chloride, lauryltrimethylammonium chloride and behenyltrimethylammonium chloride); alkylpyridinium salt (e.g. cetylpyridinium chloride); distearyldimethylammonium chloride, dialkyldimethylammonium salt; poly-(N,N'-dimethyl-3,5-methylenepiperidinium) chloride; alkyl quaternary ammonium salt; alkyldimethylbenzylammonium salt; alkyl isoquinolinium salt; dialkyl morpholinium salt; POE alkylamine; alkylamine salt; polyamine fatty acid derivative; amyl alcohol fatty acid derivative; benzalkonium chloride; and benzethonium chloride.

Examples of powder components include inorganic powder (e.g talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, metallic tungstate, magnesium, silica, zeolite, barium sulfate, calcined calcium sulfate (calcined plaster), calcium phosphate, fluorine apatite, hydroxyapatite, ceramic powder, metallic soap (e.g. zinc myristate, calcium palmitate and aluminum stearate) and boron nitride); organic powder (e.g. polyamide resin powder (nylon powder), polyethylene powder, polymethylmethacrylate powder, polystyrene powder, styrene/acrylic acid copolymer resin powder, benzoguanamine resin powder, polytetrafluoroethylene powder, and cellulose powder); inorganic white pigment (e.g. titanium oxide and zinc oxide); inorganic red pigment (e.g. iron oxide (red iron oxide) and iron titanate); inorganic brown pigment (e.g. gamma-iron oxide); inorganic yellow pigment (e.g. yellow iron oxide and yellow ocher); inorganic black pigment (e.g. black iron oxide and low-order titanium oxide); inorganic violet pigment (e.g. mango violet and cobalt violet); inorganic green pigment (e.g. chromic oxide, chromium hydroxide and cobalt titanate); inorganic blue pigment (e.g. ultramarine and Prussian blue); pearl pigment (e.g. titanium oxide-coated mica, titanium oxide-coated bismuth oxychloride, titanium oxide-coated talc and colored titanium oxide-coated mica, bismuth oxychloride and argentine); metallic powder pigment (e.g. aluminum powder and copper powder); organic pigment such as zirconium lake, barium lake and aluminum lake (e.g. organic pigment such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, Blue No. 404, Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3 and Blue No. 1); and natural colorant (e.g. chlorophyll and beta-carotene).

Examples of natural water-soluble polymers include plant polymer (e.g. gum arabic, tragacanth gum, galactan, guar gum, carob gum, sterculia urens gum, carrageenan, pectin, agar, quince seed (marmelo), algal colloid (brown alga extract), starch (rice, corn, popate and wheat) and glycyrrhizic acid); microbial polymer (e.g. xanthane gum, dextran, succinoglycan and pullulan); and animal polymer (e.g. collagen, casein, albumin and gelatin). Also, their derivatives (POE/POP modified, alkyl modified, cationized, anionized or silylated derivatives) may be included.

Examples of semisynthetic water-soluble polymers include starch polymer (e.g. carboxymethyl starch and methylhydroxypropyl starch); cellulose polymer (methyl cellulose, ethyl cellulose, methylhydroxypropyl cellulose, hydroxyethyl cellulose, cellulose sulfate sodium salt, dialkyldimethylammonium sulfate cellulose, hydroxypropyl cellulose, carboxymethyl cellulose, sodium carboxymethyl cellulose, crystalline cellulose, cellulose powder, hydrophobic modified compounds (e.g. partially stearoxy-modified compound) of these polymer, and cationized compounds of these polymer); alginic acid polymer (e.g. sodium alginate and alginic acid propylene glycol ester); and sodium pectate.

Examples of synthetic water-soluble polymers include vinyl polymer (e.g. polyvinyl alcohol, polyvinylmethylether, polyvinylpyrrolidone and carboxy vinyl polymer); polyoxyethylene polymer (e.g. polyoxyethylene/polyoxypropylene copolymer with polyethylene glycol 20,000, 40,000 or 60,000); poly-(dimethyldiallylammonium halide) type cationic polymer (e.g. Merquat 100 manufactured by Merck & Co., Inc.); dimethyldiallylammonium halide/acrylamide copolymer type cationic polymer (e.g. Merquat 550 manufactured by Merck & Co., Inc.); acrylic polymer (e.g. sodium polyacrylate, polyethylacrylate and polyacrylamide); polyethyleneimine; cationic polymer; magnesium aluminum silicate (Veegum); Polyquaternium-39; polyquaternium-47; and polyquaternium-74; propyltrimonium chloride acrylamide/dimethylacrylamide copolymer.

Examples of thickeners include gum arabic, carrageenan, sterculia urens gum, tragacanth gum, carob gum, quince seed (marmelo), casein, dextrin, gelatin, sodium pectate, sodium alginate, methyl cellulose, ethyl cellulose, CMC, hydroxyethyl cellulose, hydroxypropyl cellulose, PVA, PVM, PVP, sodium polyacrylate, carboxy vinyl polymer, locust bean gum, guar gum, tamarind gum, dialkyldimethylammonium sulfate cellulose, xanthane gum, magnesium aluminum silicate, bentonite, hectorite, magnesium aluminum silicate (Veegum), laponite and silicic anhydride.

Examples of ultraviolet absorbers include benzoic acid-based ultraviolet absorber (e.g. para-aminobenzoic acid (hereinafter abbreviated as PABA), PABA monoglycerin ester, N,N-dipropoxy PABA ethyl ester, N,N-diethoxy PABA ethyl ester, N,N-dimethyl PABA ethyl ester and N,N-dimethyl PABA butyl ester); anthranilic acid-based ultraviolet absorber (e.g. homomentyl-N-acetylanthranilate); salicylic acid-based ultraviolet absorber (e.g. amyl salicylate, mentyl salicylate, homomentyl salicylate, octyl salicylate, phenyl salicylate, banzyl salicylate and p-isopropanolphenyl salicylate); cinnamic acid-based ultraviolet absorber (e.g. octyl cinnamate, ethyl-4-isopropyl cinnamate, methyl-2,5-diisopropyl cinnnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, octyl-p-methoxycinnamate (2-ethylhexyl-p-methoxycinnamate), 2-ethoxyethyl-p-methoxycinnamate, cyclohexyl-p-methoxycinnamate, ethyl alpha-cyano-beta-phenylcinnamate, 2-ethylhexyl alpha-cyano-beta-phenylcinnamate; glyceryl mono-2-ethylhexanoyl-diparamethoxycinnamate); benzophenone-based ultraviolet absorber (e.g. 2,4-dihydroxybenzophenone, 2,2'-dihydroxy-4-methoxybenzophenone, 2,2'-dihydroxy-4,4'-dimethoxybenzophenone, 2,2'-4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone); 3-(4'-methylbenzylidene)-d,1-camphor, 3-benzylidene-d,1-camphor; 2-phenyl-5-methylbenzoxazole; 2,2-hydroxy-5-methylphenylbenzotriazole; 2-(2'-hydroxy-5'-t-octylphenyl) benzortiazole; 2-(2'-hydroxy-5'-methylphenylbenzotriazole; dianysoylmethane; 4-methoxy-4'-t-butyldibenzoylmethane; 5-(3,3-dimethyl-2-norbornilidene)-3-pentan-2-one; and triazine-based ultraviolet absorber (e.g. 2-4-[(2-hydroxy-3-dodecyloxypropyl)oxy]-2-hydoxyphenyl)-4,6-bis(2,4-dimethylphenyl)-1,3, 5-triazine, 2-4-[(2-hydroxy-3-tridecyloxypropyl)oxy]-2-hydroxyphenyl-4,6-bis(2,4-dimethylphenyl)-1,3, 5-triazine).

Examples of sequestrants include 1-hydroxyethane-1,1-diphosphonate, tetrasodium 1-hydroxyethane-1,1-diphosphonate, edetate disodium, edetate trisodium, edetate tetrasodium, sodium citrate, sodium polyphosphate, sodium metaphosphate, gluconic acid, phosphoric acid, citric acid, ascorbic acid, succinic acid, edetic acid, trisodium (hydroxyethyl) ethylenediamine triacetate.

Examples of pH adjusters include buffering agents such as lactic acid/sodium lactate, citric acid/sodium citrate, succinic acid/sodium succinate.

Examples of vitamins include vitamin A, B1, B2, B6, C, E and derivatives thereof, pantothenic acid and derivatives thereof, and biotin.

Examples of antioxidants include tocopherol, dibutylhydroxytoluene, butylhydroxyanisol and gallic acid ester.

Examples of other usable components include preservatives (e.g. ethylparaben, butylparaben, 1,2-alkanediol (having a carbon chain length of 6 to 14) and derivatives thereof, phenoxyethanol and methylchloroisothiazolinone); antiphlogistics (e.g. glycyrrhizic acid derivatives, glycyrrhetinic acid derivatives, salicylic acid derivatives, hinokitiol, zinc oxide and allantoin); whitening agents (e.g. saxifrage extract and arbutin); various extracts (e.g. phellodendron bark, coptis rhizome, lithospermum, peony, swertia herb, birch, sage, loquat, carrot, aloe, mallow, iris, grapes, coix seed, dishcloth gourd, lily, saffron, cnidium rhizome, ginger, Saint John's wort, ononis, garlic, capsicum, citrus unshiu peel, angelica acutiloba and seaweed); activator agents (royal jelly, photosensitive principle and cholesterol derivatives); blood circulation accelerators (e.g. nonanoic acid vanillylamide, benzyl nicotinate, beta-butoxyethyl nicotinate, capsaicin, zingerone, cantharidis tincture, ichthammol, tannic acid, alpha-borneol, tocopherol nicotinate, inositol hexanicotinate, cyclandelate, cinnarizin, tolazoline, acetylcholine, verapamil, cepharanthine and gamma-oryzanol); antiseborrheic agents (e.g. sulfur and thianthol); anti-inflammatory agents (e.g. tranexamic acid, thiotaurine and hypotaurine); and aromatic alcohols (e.g. benzyl alcohol and benzyloxyethanol).

The concentrated cleanser composition of the present invention can be used for hair shampoo, body cleanser, facial cleanser, baby shampoo, baby body cleanser, kitchen cleanser, medical cleanser, and various other applications involving cleanser compositions, and the respective usage forms are not limited in particular.

### EXAMPLES

Hereinafter, the present invention will be explained in further detail with reference to examples. However, the present invention is not limited by these examples. Unless otherwise stated, the blending quantities are all expressed by mass %.

At first, the evaluation methods for the concentrated cleanser composition (and its diluted material) used in the present examples will be explained.

### Evaluation method for concentrated cleanser composition (and its diluted material)

The viscosities of the concentrated cleanser composition (and its diluted material) were respectively measured, with a B-type viscometer, as the values after rotating for 1 minute.

The ease of dilution into water was evaluated from the ease of mixing by agitation when water was added to the concentrated cleanser composition; the dilution was carried out so that the concentration of mixed anionic and amphoteric surfactants was 15 mass %. Specifically, a predetermined amount of concentrated cleanser composition and water were mixed in a screw cap test tube, and it was judged by the number of times of light shaking. The "evaluation during dilution" was represented as follows:

If no drastic viscosity increase was observed during dilution and the dilution could be carried out very easily, it was designated as "A".

If no drastic viscosity increase was observed during dilution and the dilution could be carried out easily, it was designated as "B".

If a drastic viscosity increase was observed during dilution though the dilution was possible, it was designated as "C".

As the "evaluation of post-dilution solution appearance", the above-described post-dilution cleanser composition (diluted material) was observed visually, and the phase state of the post-dilution solution was evaluated.

In order to study the blending of an anionic surfactant and an amphoteric surfactant, the above-described evaluations were carried out for the formulations listed in Table 1 below. The results are shown in Table 1. In the table, the values shown in the parentheses represent the net amounts of the surfactants.

**Table 1**

| | Test Example 1-1 | Test Example 1-2 | Test Example 1-3 | Test Example 1-4 |
|---|---|---|---|---|
| <Anionic surfactant> | | | | |
| 27% polyoxyethylene sodium lauryl sulfate solution (Alscope NS-230, manufactured by Toho Chemical Industry Co., Ltd.) | 38.5 (10.4) | -- | -- | -- |
| 70% polyoxyethylene(2) sodium lauryl sulfate solution (Texapon N70, manufactured by Cognis Corporation) | -- | 38.5 (27.0) | 77.0 (53.9) | -- |

| <Amphoteric surfactant> | | | | |
|---|---|---|---|---|
| 40% lauryl dimethyl amino acetate betaine solution (Anon BL-SF, manufactured by NOF Corporation) | 38.5 (15.4) | 38.5 (15.4) | -- | 77.0 (30.8) |

| <Alcohol> | | | | |
|---|---|---|---|---|
| Dipropylene glycol | 11.5 | 11.5 | 11.5 | 11.5 |

| <Nonionic surfactant> | | | | |
|---|---|---|---|---|
| Diethylene glycol laurate | 11.5 | 11.5 | 11.5 | 11.5 |
| Anionic surfactant + Amphoteric surfactant (Net) | 25.8 | 42.4 | 53.9 | 30.8 |
| Pre-dilution viscosity [mPa·s] | 100 | 6650 | 25000 | 50 |
| Evaluation during dilution | A | A | C | A |
| Post-dilution viscosity [mPa·s] | 160 | 1300 | 10 | 5 |
| Dilution rate | 1.72 | 2.82 | 3.59 | 2.05 |
| Post-dilution solution appearance | One phase | One phase | One phase | One phase |

### (Production method)

Into an anionic surfactant solution, dipropylene glycol and a nonionic surfactant were mixed with stirring, and then an amphoteric surfactant solution was added and mixed; thus a concentrated liquid cleanser composition was obtained.

As shown in Table 1, Test Example 1-2, wherein an anionic surfactant and an amphoteric surfactant were combined and blended in the net amount of 40 mass % or more, was excellent in the ease of dilution, and the post-dilution viscosity was suitable to cleansers (300 to 20000 mPa • s).

On the other hand, in Test Example 1-1, wherein the concentrations of an anionic surfactant and an amphoteric surfactant were lowered, and in Test Examples 1-3 and 1-4, wherein only an anionic surfactant or an amphoteric surfactant was blended, the viscosity increase during dilution and the viscosity decrease of the post-dilution composition were significant.

Based on the above, it is preferable in the present invention to contain the total net amount of 40 mass % or more of an anionic surfactant and an amphoteric surfactant.

In order to study the blending of an alcohol and a cosurfactant (nonionic surfactant), the above-described evaluations were carried out for the formulations listed in Table 2 below. The results are shown in Table 2. In the table, the values shown in the parentheses represent the net amounts of the surfactants.

**Table 2**

| | Test Example 2-1 | Test Example 2-2 | Test Example 2-3 | Test Example 2-4 | Test Example 2-5 | Test Example 2-6 |
|---|---|---|---|---|---|---|
| | | | | | | |
| 70% polyethylene(2) sodium lauryl sulfate solution (Texapon N70, manufactured by Cognis Corporation) | 38.5 (27.0) | 38.5 (27.0) | 38.5 (27.0) | 38.5 (27.0) | 38.5 (27.0) | 38.5 (27.0) |

| <Amphoteric surfactant> | | | | | | |
|---|---|---|---|---|---|---|
| 40% lauryl dimethyl amino acetate betain solution (Anon BL-SF, manufactured by NOF Corporation) | 38.5 (15.4) | 38.5 (15.4) | 38.5 (15.4) | 38.5 (15.4) | 38.5 (15.4) | 38.5 (15.4) |

| <Alcohol> | | | | | | |
|---|---|---|---|---|---|---|
| Dipropylene glycol | 23.0 | 18.0 | 13.0 | 9.0 | 7.0 | -- |

| <Nonionic surfactant> | | | | | | |
|---|---|---|---|---|---|---|
| Diethylene glycol laurate | -- | 5.0 | 10.0 | 14.0 | 16.0 | 23.0 |
| Anionic surfactant + Amphoteric surfactant | 42.4 | 42.4 | 42.4 | 42.4 | 42.4 | 42.4 |
| Pre-dilution viscosity [mPa•s] | 90 | 850 | 1250 | 7500 | 18000 | 122000 |
| Evaluation during dilution | A | A | A | B | B | C |
| Post-dilution viscosity [mPa•s] | 15 | 15 | 405 | 15900 | 16800 | 14200 |
| Dilution rate | 2.82 | 2.82 | 2.82 | 2.82 | 2.82 | 2.82 |
| Post-dilution solution appearance | One phase | One phase | One phase | One phase | One phase | Two phases |

### (Production method)

Into an anionic surfactant solution, an alcohol and a nonionic surfactant were mixed with stirring, and then an amphoteric surfactant solution was added and mixed; thus a concentrated liquid cleanser composition was obtained.

As shown in Table 2, in Test Example 2-1 and Test Example 2-6, wherein either dipropylene glycol or diethylene glycol laurate was blended, the viscosity of the composition became significantly low or became significantly high. Among Test Examples 2-2 to 2-5, wherein dipropylene glycol and diethylene glycol laurate were used in combination, in Test Example 2-2, wherein a large amount of dipropylene glycol was blended, the evaluation of the post-dilution viscosity was not good.

On the other hand, in Test Examples 2-3 to 2-5, wherein suitable amounts of dipropylene glycol and diethylene glycol laurate were blended, good results in all the evaluation items were obtained.

Based on the above, it is preferable to blend, in the present invention, suitable amounts of an alcohol and a nonionic surfactant in combination.

As a result of further testing, in the present invention, when the blending quantity of an alcohol is in the range of 5 to 15 mass %, the blending quantity of a nonionic surfactant is 8 to 18 mass %, and the blending ratio of the two components (alcohol : nonionic surfactant) is 3.5:1 to 1:2.5, a concentrated liquid cleanser composition excellent in all the evaluations could be obtained.

In order to study the blending of an alcohol, the above-described evaluations were carried out for the formulations listed in Table 3 below. The results are shown in Table 3. In the table, the values shown in the parentheses represent the net surfactant amounts.

**Table 3**

| | Test Example 3-1 | Test Example 3-2 | Test Example 3-3 |
|---|---|---|---|
| <Anionic surfactant> | | | |
| 70% polyethylene(2) sodium lauryl sulfate solution (Texapon N70, manufactured by Cognis Corporation) | 38.5 (27.0) | 38.5 (27.0) | 38.5 (27.0) |

| <Amphoteric surfactant> | | | |
|---|---|---|---|
| 40% lauryl dimethyl amino acetate betain solution (Anon BL-SF, manufactured by NOF Corporation) | 38.5 (15.4) | 38.5 (15.4) | 38.5 (15.4) |

| <Alcohol> | | | |
|---|---|---|---|
| Dipropylene glycol | 11.0 | -- | -- |
| Ethanol | -- | 11.0 | |
| Glycerin | -- | -- | 11.0 |

| <Nonionic surfactant> | | | |
|---|---|---|---|
| Diethylene glycol laurate | 12.0 | 12.0 | 12.0 |
| Anionic surfactant + Amphoteric surfactant | 42.4 | 42.4 | 42.4 |
| Pre-dilution viscosity [mPa•s] | 11550 | 3600 | 10400 |
| Evaluation during dilution | A | A | B |
| Post-dilution viscosity [mPa•s] | 2550 | 2000 | 20650 |
| Dilution rate | 2.82 | 2.82 | 2.82 |
| Post-dilution solution appearance | One phase | One phase | One phase |

### (Production method)

Into an anionic surfactant solution, an alcohol and a nonionic surfactant were mixed with stirring, and then an amphoteric surfactant solution was added and mixed; thus a concentrated liquid cleanser composition was obtained.

As shown in Table 3, Test Examples 3-1 and 3-2, wherein dipropylene glycol (dihydric alcohol) and ethanol (monohydric alcohol) were blended, were excellent in the ease of dilution, and the post-dilution viscosity was suitable to cleansers (300 to 20000 mPa • s).

On the other hand, in Test Example 3-3, wherein glycerin (trihydric alcohol) was used, the post-dilution viscosity was high, and the handling was difficult at the time of use.

Based on the above, in the present invention, it is preferable to use a monohydric or dihydric alcohol.

In order to study the blending of a nonionic surfactant, the above-described evaluations were carried out for the formulations listed in Table 4 and Table 5 below. The results are shown in Table 4 and Table 5. In the tables, the values shown in the parentheses represent the net amounts of the surfactants.

**Table 4**

| | Test Example 4-1 | Test Example 4-2 | Test Example 4-3 | Test Example 4-4 | Test Example 4-5 |
|---|---|---|---|---|---|
| <Anionic surfactant> | | | | | |
| 70% polyoxyethylene(2) sodium lauryl sulfate solution (Texapon N70, manufactured by Cognis Corporation) | 38.5 (27.0) | 38.5 (27.0) | 38.5 (27.0) | 38.5 (27.0) | 38.5 (27.0) |

| <Amphoteric surfactant> | | | | | |
|---|---|---|---|---|---|
| 40% lauryl dimethyl amino acetate betaine solution (Anon BL-SF, manufactured by NOF Corporation) | 38.5 (15.4) | 38.5 (15.4) | 38.5 (15.4) | 38.5 (15.4) | 38.5 (15.4) |

| <Alcohol> | | | | | |
|---|---|---|---|---|---|
| Dipropylene glycol | 11.5 | 11.5 | 11.5 | 11.5 | 11.5 |

| <Nonionic surfactant> | | | | | |
|---|---|---|---|---|---|
| Diethylene glycol laurate (IOB: 0.89, Mw = 302) | 11.5 | -- | -- | -- | 6 |
| Coconut oil fatty acid N-methylethanolamide (IOB: 0.94, Mw = 277) | -- | 11.5 | -- | -- | -- |
| Propylene glycol laurate (IOB: 0.52, Mw = 272) | -- | -- | 11.5 | -- | 5.5 |
| Coconut oil fatty acid diethanolamide (IOB: 1.18, Mw = 307) | -- | -- | -- | 11.5 | -- |
| Anionic surfactant + Amphoteric surfactant | 42.4 | 42.4 | 42.4 | 42.4 | 42.4 |
| Pre-dilution viscosity [mPa•s] | 6650 | 3850 | 12000 | 68000 | 12650 |
| Evaluation during dilution | A | A | A | C | A |
| Post-dilution viscosity [mPa•s] | 1300 | 600 | 4000 | 255 | 5650 |
| Dilution rate | 2.82 | 2.82 | 2.82 | 2.82 | 2.82 |
| Post-dilution solution appearance | One phase | One phase | Two phases | One phase | One phase |

**Table 5**

| | Test Example 4-6 | Test Example 4-7 | Test Example 4-8 |
|---|---|---|---|
| <Anionic surfactant> | | | |
| 70% polyoxyethylene(2) sodium lauryl sulfate solution (Texapon N70, manufactured by Cognis Corporation) | 38.5 (27.0) | 38.5 (27.0) | 38.5 (27.0) |

| <Amphoteric surfactant> | | | |
|---|---|---|---|
| 40% lauryl dimethyl amino acetate betaine solution (Anon BL-SF, manufactured by NOF Corporation) | 38.5 (15.4) | 38.5 (15.4) | 38.5 (15.4) |

| <Alcohol> | | | |
|---|---|---|---|
| Dipropylene glycol | 11.5 | 11.5 | 11.5 |

| <Nonionic surfactant> | | | |
|---|---|---|---|
| Pluronic L64 (IOB: 0.98, Mw = 2860) | 11.5 | -- | -- |
| Oleic acid (IOB: 0.42, Mw = 282) | -- | 11.5 | -- |
| POE(2) coconut oil fatty acid monoethanolamide (IOB: 1.16, Mw = 351) | | | 11.5 |
| Anionic surfactant + Amphoteric surfactant | 42.4 | 42.4 | 42.4 |
| Pre-dilution viscosity [mPa•s] | 4050 | 3450 | 100000 |
| Evaluation during dilution | A | A | C |
| Post-dilution viscosity [mPa•s] | 15 | 100 | 30 |
| Dilution rate | 2.82 | 2.82 | 2.82 |
| Post-dilution solution appearance | One phase | Two phases | One phase |

| | | | |
|---|---|---|---|
| Mw: average molecular weight | | | |

### (Production method)

Into an anionic surfactant solution, an alcohol and a nonionic surfactant were mixed with stirring, and then an amphoteric surfactant solution was added and mixed; thus a concentrated liquid cleanser composition was obtained.

As shown in Table 4 and Table 5, in Test Examples 4-1 and 4-2, wherein a nonionic surfactant with the IOB value of 0.8 to 1.1 was blended, a concentrated cleanser composition excellent in the degree of viscosity change during dilution and excellent in the post-dilution viscosity was obtained.

On the other hand, in Test Examples 4-3 and 4-7, wherein a nonionic surfactant with the IOB value of less than 0.8 was blended, the solution became two-phase after dilution. In Test Examples 4-4 and 4-8, wherein a nonionic surfactant with the IOB value higher than 1.1 was blended, a concentrated cleanser composition excellent in the degree of viscosity change during dilution and excellent in the post-dilution viscosity was not obtained.

In Test Example 4-5, wherein a nonionic surfactant with the IOB value of 0.8 to 1.1 and a nonionic surfactant with another IOB value were used in combination, the appearance of the post-dilution solution turned out to be good.

In Test Example 4-6, wherein a high-molecular-weight long-chain nonionic surfactant was blended, the post-dilution composition viscosity was significantly low even when the IOB value was in the range of 0.8 to 1.1.

Based on the above, in the present invention, it is preferable to blend a nonionic surfactant with the IOB value of 0.8 to 1.1, wherein neither the hydrophobic group nor the hydrophilic group has a long chain. As a result of further investigation, it was found that the molecular weight of the nonionic surfactant was preferably 500 or lower.

Furthermore, the below-described evaluations were carried out in addition to the above-described evaluations. In order to study the blending of a salt, the above-described evaluations were carried out for the formulations listed in Table 6 and Table 7 below. In the tables, the values shown in the parentheses represent the net amounts of the surfactants.

In Table 6 below, Test Examples 5-2, 5-3, 5-4, 5-5, and 5-6 were respectively obtained by diluting the composition in Test Example 5-1 with water 1.5 times, 2.0 times, 2.5 times, 2.82 times, and 3 times.

Similarly, in Table 7 below, Test Examples 5-8, 5-9, 5-10, 5-11, and 5-12 were respectively obtained by diluting the composition of Test Example 5-7 with water 1.5 times, 2.0 times, 2.5 times, 2.82 times, and 3 times.

**Table 6**

| | Test Example 5-1 | Test Example 5-2 | Test Example 3-3 | Test Example 5-4 | Test Example 5-5 | Test Example 5-6 |
|---|---|---|---|---|---|---|
| <Anionic surfactant> | | | | | | |
| 70% polyoxyethylene(2) sodium lauryl sulfate solution (Texapon N70, manufactured by Cognis Corporation) | 38.5 (27.0) | 25.7 (18.0) | 19.3 (13.5) | 15.4 (10.8) | 13.7 (9.6) | 12.8 (9.0) |

| <Amphoteric surfactant> | | | | | | |
|---|---|---|---|---|---|---|
| 40% lauryl dimethyl amino acetate betaine solution (Anon BL-SF, manufactured by NOF Corporation) | 38.5 (15.4) | 25.7 (10.3) | 19.3 (7.7) | 15.4 (6.2) | 13.7 (5.5) | 12.8 (5.1) |

| <Alcohol> | | | | | | |
|---|---|---|---|---|---|---|
| Dipropyleneglycol | 11.5 | 7.67 | 5.75 | 4.60 | 4.08 | 3.83 |

| <Nonionic surfactant> | | | | | | |
|---|---|---|---|---|---|---|
| Diethylene glycol laurate | 11.5 | 7.67 | 5.75 | 4.60 | 4.08 | 3.83 |

| <Salt> | | | | | | |
|---|---|---|---|---|---|---|
| Ammonium chloride | 0 | 0 | 0 | 0 | 0 | 0 |

| <Water> | | | | | | |
|---|---|---|---|---|---|---|
| Water | Balance | Balance | Balance | Balance | Balance | Balance |
| Anionic surfactant + Amphoteric surfactant | 42.4 | 28.3 | 21.2 | 17.0 | 15.1 | 14.1 |
| Viscosity [mPa•s] | 6650 | 700 | 2250 | 2500 | 1300 | 700 |
| Dilution solution appearance (during dilution and post-dilution) | -- | One phase | One phase | One phase | One phase | One phase |

**Table 7**

| | Test Example 5-7 | Test Example 5-8 | Test Example 5-9 | Test Example 5-10 | Test Example 5-11 | Test Example 5-12 |
|---|---|---|---|---|---|---|
| <Anionic surfactant> | | | | | | |
| 70% polyoxyethylene(2) sodium lauryl sulfate solution (Texapon N70, manufactured by Cognis Corporation) | 38.5 (27.0) | 25.7 (18.0) | 19.3 (13.5) | 15.4 (10.8) | 13.7 (9.6) | 12.8 (9.0) |

| <Amphoteric surfactant> | | | | | | |
|---|---|---|---|---|---|---|
| 40% lauryl dimethyl amino acetate betaine solution (Anon BL-SF, manufactured by NOF Corporation) | 38.5 (15.4) | 25.7 (10.3) | 19.3 (7.7) | 15.4 (6.2) | 13.7 (5.5) | 12.8 (5.1) |

| <Alcohol> | | | | | | |
|---|---|---|---|---|---|---|
| Dipropylene glycol | 11.5 | 7.67 | 5.75 | 4.60 | 4.08 | 3.83 |

| <Nonionic surfactant> | | | | | | |
|---|---|---|---|---|---|---|
| Diethylene glycol laurate | 10.3 | 6.87 | 5.15 | 4.12 | 3.65 | 3.43 |

| <Salt> | | | | | | |
|---|---|---|---|---|---|---|
| Ammonium chloride | 1.2 | 0.8 | 0.6 | 0.48 | 0.43 | 0.4 |

| <Water> | | | | | | |
|---|---|---|---|---|---|---|
| Water | Balance | Balance | Balance | Balance | Balance | Balance |
| Anionic surfactant + Amphoteric surfactant | 42.4 | 28.3 | 21.2 | 17.0 | 15.1 | 14.1 |
| Viscosity [mPa•s] | 3000 | 350 | 600 | 1400 | 3600 | 3350 |
| Dilution solution appearance (during dilution and post-dilution) | - | One phase | One phase | One phase | One phase | One phase |

### (Production method)

Into an anionic surfactant solution, an alcohol and a nonionic surfactant were mixed with stirring, and then a salt and an amphoteric surfactant solution were added and mixed; thus a concentrated liquid cleanser composition was obtained.

As shown in Tables 6 and 7, Test Example 5-7, wherein a salt was blended, was excellent in that the viscosity difference before and after dilution was small and the dilution was easy, because of the blending of a salt, compared with Test Example 5-1, wherein a salt was not blended. In addition, a composition with moderately high post-dilution viscosity was obtained by the blending of a salt regardless of low viscosity before dilution and during dilution.

Based on the above, it is preferable to further blend a salt in the present invention.

Hereinafter, the formulation examples of the present invention are shown; however, the present invention is not limited by these examples.

### <Prescription example 1: concentrated hair shampoo>

| **(Component)** | **(% by mass)** |
|---|---|
| 70% polyoxyethylene(2) sodium lauryl sulfate solution (Texapon N70 manufactured by Cognis Corporation) | 20.0 |
| 70% ammonium lauryl sulfate solution (Texapon ALS70 manufactured by Cognis Corporation) | 15.0 |
| Sodium methyl lauroyl taurate | 1.8 |
| 39% cocamide propyl betaine solution (Dehyton PK45 manufactured by Cognis Corporation, containing 6% sodium chloride) | 37.0 |
| Coconut oil fatty acid N-methylethanolamide | 8.3 |
| Coconut oil fatty acid monoethanolamide | 0.5 |
| Dipropylene glycol | 12.0 |
| Sorbitol | 1.5 |
| Polyquaternium-7 (Merquat 2200 manufactured by Nalco Company) | 0.2 |
| Polyquaternium-10 | 0.1 |
| Citric acid | 0.5 |
| Sodium citrate | 0.5 |
| Bis-isobutyl PEG-14/amodimethicone copolymer | 0.1 |
| Myristyl alcohol | 0.5 |
| Menthol | 0.4 |
| Phenoxyethanol | 0.8 |
| POP(70) decaglyceryl ether (Beltamol DG-25 manufactured by NOF Corporation) | 0.1 |
| Perfume | 0.7 |

### (Production method)

Into polyoxyethylene(2) sodium lauryl sulfate solution and an ammonium lauryl sulfate solution, coconut oil fatty acid N-methylethanolamide, coconut oil fatty acid monoethanolamide, and dipropylene glycol are mixed with stirring. The composition is obtained by further mixing other components.

The obtained composition can be easily diluted in water, and it can suitably be used as hair shampoo by diluting 2.71 times with water.

### <Prescription example 2: concentrated shower gel>

| **(Component)** | **(% by mass)** |
|---|---|
| 70% polyoxyethylene(1) sodium lauryl sulfate (Sinolin SPE-1150 manufactured by New Japan Chemical Co., Ltd.) | 34.5 |
| 90% laureth-4 carboxylate (Empocol CBC manufactured by Huntsman International LLC.) | 3.0 |
| 30% ammonia solution | 0.4 |
| 40% lauryl dimethyl amino acetate betaine (Nissananon BL-SF manufactured by NOF Corporation) | 31.5 |
| 85% cocamide propyl betaine (TEGO Betain CK D manufactured by Evonik-Degussa, containing 15% sodium chloride) | 7.0 |
| Diethylene glycol laurate (manufactured by Clariant (Japan) K.K.) | 8.0 |
| POE(1) -1,2-dodecanediol | 1.0 |
| Dipropylene glycol | 12.3 |
| Cationized locust bean gum | 0.2 |
| Phenoxyethanol | 0.8 |
| Sodium benzoate | 0.2 |
| Lactic acid | 0.2 |
| PPG(70) glyceryl ether | 0.1 |
| Perfume | 0.8 |

### (Production method)

Polyoxyethylene(1) sodium lauryl sulfate, 90% laureth-4 carboxylate and ammonia solution are mixed with stirring. With this solution, diethylene glycol laurate, POE(1) -1,2-dodecanediol and dipropylene glycol are mixed with stirring. The composition is obtained by further mixing other components.

The obtained composition can be easily diluted in water, and it can suitably be used as shower gel by diluting 3.03 times with water.

### <Prescription example 3 concentrated hair shampoo>

| **(Component)** | **(% by mass)** |
|---|---|
| 90% laureth-4 carboxylate (Empocol CBC manufactured by Huntsman International LLC.) | 30.0 |
| 30% imidazolinium betaine (Obazolin 662N manufactured by Toho Chemical Industry Co., Ltd.) | 10.0 |
| 40% lauryl dimethyl amino acetate betaine (Nissananon BL-SF manufactured by NOF Corporation) | 30.0 |
| Diethylene glycol laurate (manufactured by Clariant (Japan) K.K.) | 11.1 |
| Dipropylene glycol | 6.0 |
| Ethanol | 6.0 |
| 30% ammonia solution | 4.0 |
| Cationized guar gum (Catinal CG-100S manufactured by Toho Chemical Industry Co., Ltd.) | 0.2 |
| Sodium chloride | 1.0 |
| Phenoxyethanol | 0.8 |
| POP(70) decaglyceryl ether (Beltamol DG-25 manufactured by NOF Corporation) | 0.1 |
| Perfume | 0.8 |

### (Production method)

Into laureth-4 carboxylate and ammonium solution, diethylene glycol laurate, dipropylene glycol and ethanol are mixed with stirring. The composition is obtained by further mixing other components.

The obtained composition can be easily diluted in water, and it can suitably be used as hair shampoo by diluting 2.8 times with water.

### <Prescription example 4: concentrated shower gel>

| **(Component)** | **(% by mass)** |
|---|---|
| 70% polyoxyethylene(1) sodium lauryl sulfate (Sinolin SPE-1150 manufactured by New Japan Chemical Co., Ltd.) | 28.0 |
| 70% polyoxyethylene(3) sodium lauryl sulfate (Sinolin SPE-1350 manufactured by New Japan Chemical Co., Ltd.) | 4.5 |
| 90% laureth-4 carboxylate (Empocol CBC manufactured by Huntsman International LLC.) | 3.0 |
| 30% ammonia solution | 0.4 |
| 85% cocamide propyl betaine (TEGO Betain CK D manufactured by Evonik-Degussa, containing 15% sodium chloride) | 25.0 |
| 27% sodium coconut oil fatty acid methyl taurate (ST-TS manufactured by NOF Corporation) | 13.7 |
| Diethylene glycol laurate (manufactured by Clariant (Japan) K.K.) | 7.0 |
| Coconut oil fatty acid N-methylethanolamide | 2.0 |
| Dipropylene glycol | 13.3 |
| Polyquaternium-47 (21 % solution) | 1.0 |
| Phenoxyethanol | 0.8 |
| Sodium benzoate | 0.2 |
| Lactic acid | 0.2 |
| PPG(70) glyceryl ether | 0.1 |
| Perfume | 0.8 |

### (Production method)

Polyoxyethylene(1) sodium lauryl sulfate, polyoxyethylene(3) sodium lauryl sulfate, 90% laureth-4 carboxylate and ammonia solution are mixed with stirring. With this solution, diethylene glycol laurate, coconut oil fatty acid N-methylethanolamide and dipropylene glycol are mixed with stirring. The composition is obtained by further mixing other components.

The obtained composition can be easily diluted in water, and it can suitably be used as shower gel by diluting 3.36 times with water.

### <Prescription example 5 concentrated hair shampoo>

| **(Component)** | **(% by mass)** |
|---|---|
| 70% polyoxyethylene(2) sodium lauryl sulfate solution (Texapon N70 manufactured by Cognis Corporation) | 20.0 |
| 70% ammonium lauryl sulfate solution (Texapon ALS70 manufactured by Cognis Corporation) | 15.0 |
| Sodium methyl lauroyl taurate | 1.8 |
| 39% cocamide propyl betaine solution (Dehyton PK45 manufactured by Cognis Corporation, containing 6% sodium chloride) | 37.0 |
| Coconut oil fatty acid N-methylethanolamide | 7.5 |
| Coconut oil fatty acid monoethanolamide | 0.5 |
| Dipropylene glycol | 13.0 |
| Sorbitol | 0.5 |
| propyltrimonium chloride acrylamide/dimethylacrylamide copolymer (20% aqueaou solution) | |
| | 1.0 |
| Polyquaternium-10 | 0.1 |
| Citric acid | 0.5 |
| Sodium citrate | 0.5 |
| Bis-isobutyl PEG-14/amodimethicone copolymer | 0.1 |
| Myristyl alcohol | 0.5 |
| Menthol | 0.4 |
| Phenoxyethanol | 0.8 |
| POP(70) decaglyceryl ether (Beltamol DG-25 manufactured by NOF Corporation) | 0.1 |
| Perfume | 0.7 |

### (Production method)

Into polyoxyethylene(2) sodium lauryl sulfate solution and ammonium lauryl sulfate solution, coconut oil fatty acid N-methylethanolamide, coconut oil fatty acid monoethanolamide and dipropylene glycol are mixed with stirring. The composition is obtained by further mixing other components.

The obtained composition can be easily diluted in water, and it can suitably be used as hair shampoo by diluting 2.71 times with water.

## Claims

1. A concentrated liquid cleanser composition, comprising:
(A) an anionic surfactant,
(B) an amphoteric surfactant,
(C) 5 to 15 mass % of a monohydric or dihydric alcohol,
(D) 8 to 18 mass % of a nonionic surfactant with the IOB value of 0.8 to 1.1 and the molecular weight of 500 or lower, and
(E) 45 mass % or less of water,
wherein the sum of (A) and (B) is 40 to 60 mass %;
wherein the blending ratio (C):(D) is 3.5:1 to 1:2.5; and
wherein the viscosity at 30 °C is 300 mPa • s or higher when the composition is diluted until the concentration of (A) and (B) becomes 15 mass %.

2. The concentrated liquid cleanser composition according to claim 1, wherein (D) the nonionic surfactant is a long-chain fatty acid N-methylethanolamide with the average number of carbon atoms of 10 to 14 and/or a diethylene glycol long-chain fatty acid ester with the average number of carbon atoms of 10 to 14.

3. The concentrated liquid cleanser composition according to claim 1 or 2, wherein (A) the anionic surfactant comprises a polyoxyethylene alkyl ether sulfate salt.

4. The concentrated liquid cleanser composition according to any of claims 1 to 3, comprising an organic or inorganic salt.

5. A method of use of the concentrated liquid cleanser composition according to any of claims 1 to 4, mixing the composition with water.

6. A production method of the concentrated liquid cleanser composition according to any of claims 1 to 4, comprising:
mixing (C) the monohydric or dihydric alcohol and (D) the nonionic surfactant into an aqueous solution of (A) the anionic surfactant; and
subsequently mixing an aqueous solution of (B) the amphoteric surfactant thereinto.

7. A production method of a liquid cleanser composition, mixing the concentrated liquid cleanser composition according to any of claims 1 to 4 with water.
